# EUROPEAN PATENT SPECIFICATION

(11) **EP 1 105 165 B1**
(45) Date of publication and mention of the grant of the patent: **27.02.2008**
(21) Application number: 99943794.0
(22) Date of filing: 20.08.1999
(51) Int. Cl.: A61L 2/18, B65B 55/10, B05C 1/08

(54) **APPARATUS AND METHOD FOR STERILIZING AN ASEPTIC WEB**
APPARATUR UND METHODE ZUM STERILISIEREN EINER ASEPTISCHEN STOFFBAHN
APPAREIL ET PROCEDE DE STERILISATION D'UNE BANDE ASEPTIQUE

(30) Priority: 21.08.1998 US 97462 P
(43) Date of publication of application: 13.06.2001
(73) Proprietor: Tetra Laval Holdings & Finance SA, 1009 Pully (CH)
(72) Inventor: BROLLIER, Brian, Cincinnati, OH 45244 (US); SHOWLER, Michael, F., Loveland, OH 45140 (US)
(74) Representative: Grünecker, Kinkeldey, Stockmair & Schwanhäusser Anwaltssozietät
(86) International application number: PCT/US1999/019000
(87) International publication number: WO 2000/010616

(56) References cited:
- US-A- 4 664 058

## Description

The present invention relates to a new improved method and apparatus for sterilizing paperboard material used for aseptic liquid containers commonly used for beverages. The method and apparatus, as taught herein, is especially useful for, but not necessarily limited too, gable top containers having a fitment attached thereto for pouring the liquid contents from the container.

### BACKGROUND OF THE INVENTION:

The paperboard container having a fitment, or pour spout, from which the liquid inside may be easily poured therefrom is fast becoming a standard in the milk and juice carton industry. In order to place a fitment on the typical paperboard container a hole must first be punched or cut out of the carton's surface. The presence of this hole increases the complexity of the sterilization process.

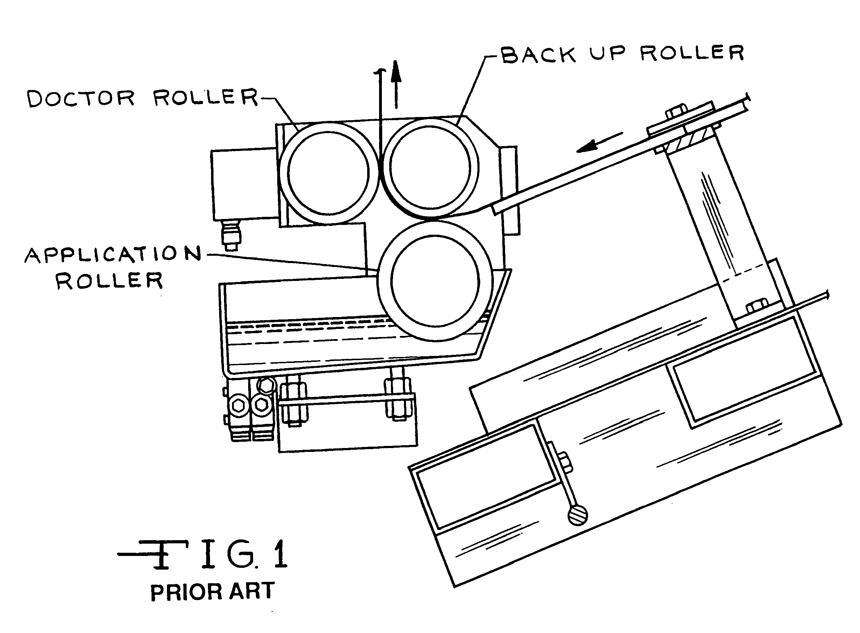

Figure 1 presents a schematic cross section of typical prior art apparatus for sterilizing paperboard material used for liquid containers. An application roller rotatingly dips into a reservoir of sterilant, typically hydrogen peroxide. The paper board stock to be sterilized is passed through a nip between the application roller and the back up roller thereby flooding the hydrogen peroxide upon the surface of the paperboard. The flooded paperboard is then passed through a nip between the back up roller and doctor roller whereby excess hydrogen peroxide is removed from the paperboard and drips back into the reservoir.

Although this process has worked well for paperboard having a continuous surface without holes therein, for the receipt of a fitment, the doctor roller/backup roller combination is unable to remove the excess sterilant that collects within the gap, or opening, presented between the doctor roll and the backup roller by the presence of the fitment hole. Thus as the sterilized paperboard is discharged from the doctor roller/backup roller nip the accumulated sterilant, within the fitment hole flows across the paperboard leaving an undesired residual of sterilant upon the board. Similarly, for paper board having surface irregularities such as score lines etc. the prior art method of sterilization causes a build up of sterilant in the areas of surface area height differentials thereby leaving globules or droplets of sterilant upon the paperboard surface. Subsequently the, system used to dry the sterilant from the board is unable to dry the residual sterilant and many times producing a sterilized paperboard having a hydrogen peroxide residual greater than 0.5 parts per million, the industry standard.

US-A-4664058 discloses a method of applying sterilant to a paper board using an application roll working in conjunction with a press roll. US-A-4233271 discloses a method utilising a rotary brush to apply a sterilant to a web of packing material.

### SUMMARY OF THE INVENTION:

The disadvantages of the prior art are overcome by the present invention as defined by claims 1 and 6. By the apparatus and method of the present invention an anilox roller is submerged in a bath of sterilant. As the surface of the anilox roller rotates upward out of the sterilant, excess sterilant is removed from the surface by a typical doctor blade. The paperboard web is then passed through a nip between the anilox roller and a backup or pressure roller. The sterilant retained within the pores of the anilox roller are thusly transferred from the anilox roller, in a printing like manner, as the paperboard web passes through the nip, and is deposited upon the paperboard thereby sterilizing the paperboard.

### BRIEF DESCRIPTION OF THE DRAWINGS:

Figure 1 shows a schematic cross section of a typical prior art machine.
Figure 2 shows a schematic cross section of the apparatus for practicing the present invention.

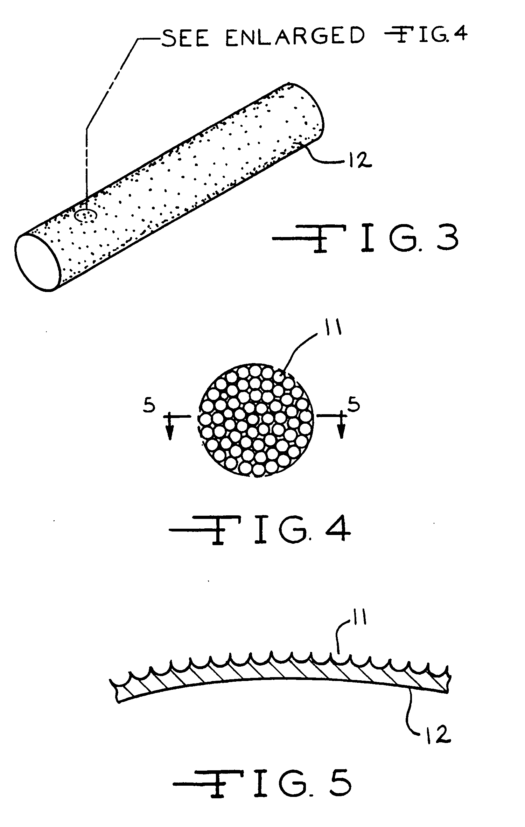

Figure 3 presents a pictorial view of a typical anilox roll suitable for practicing the present invention.
Figure 4 is a partial enlarged view of the surface of the anilox roll as shown in figure 3.
Figure 5 is a cross sectional view taken along line 5-5 of figure 4.

### PREFERRED EMBODIMENT OF THE INVENTION:

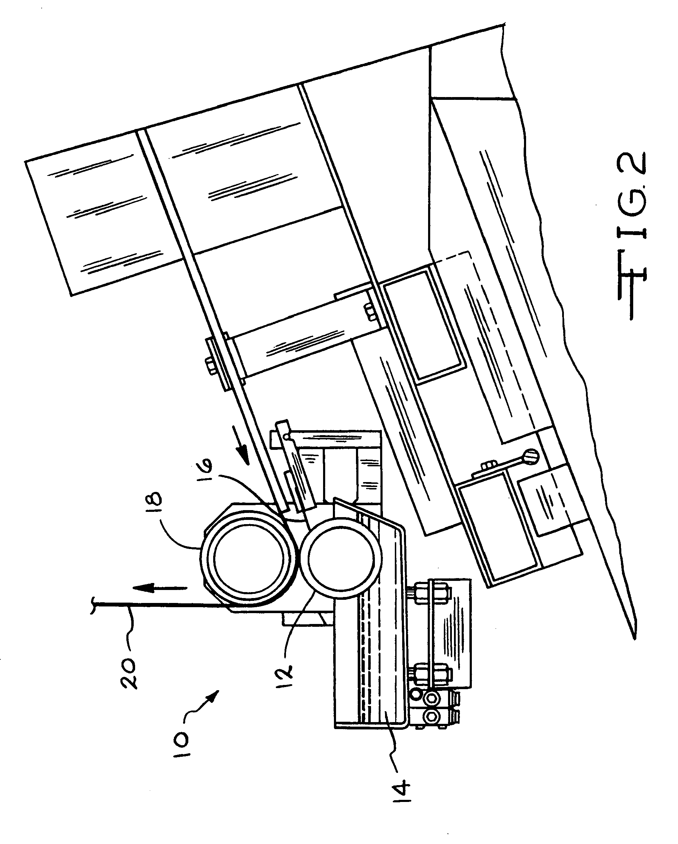

Referring now to figure 2, a cross sectional schematic is shown of the apparatus 10 suitable for the practice of the present invention. An anilox printing roller 12 is rotatingly submerged and passed through a bath of sterilant 14 where the micro pores on the cylindrical surface of the anilox roll fill with sterilant. A trailing edge doctor blade 16 wipes excess sterilant from the surface of roll 12. Paper board 20 is continuously passed through the nip between anilox roll 12 and pressure roll 18 whereby the sterilant contained within the pores 11 of roll 12 is deposited upon paperboard 20 thereby sterilizing the paperboard surface. The wetted paperboard is then passed through a drying apparatus (not shown) and on to the filling machine.

Anilox roll 12 is similar to the anilox roll as used in the rotogravure printing process. We have found that a anilox roll comprising a laser engraved, plasma coated, ceramic covered metal roll having a uniform distribution of micro cells or pores about its cylindrical surface has performed well. The surface of roll 12 has 100 percent of its cylindrical surface engraved with a uniform array of pores having a 300 line count and 6 billion cubic microns per square inch (BCM).

By use of the method and apparatus disclosed above the sterilant is uniformly deposited upon the paperboard surface only. Thus none of the sterilant is floodingly deposited in hole cut outs intended for fitment application thereby eliminating the undesirable collection of residual sterilant upon the paperboard.

## Claims

1. A method of sterilizing a paperboard material having a surface **characterized by** one or more surface irregularities in the smoothness and/or continuity thereof comprising the steps of:
a) providing a reservoir of sterilant fluid,
b) providing an anilox roll having at least a portion of its outer circumference at least partly submerged in said fluid said anilox roll having defined in the outer circumference thereof a plurality of minute discrete pores being spaced apart by respective minimal distances,
c) rotating said anilox roll within said fluid such that the pores of said anilox roll receive therein a quantity of said fluid,
d) removing from the outer circumference of said anilox roll substantially all sterilant fluid not disposed within said pores by a doctor blade,
e) thereafter, passing said paperboard material over the exposed portion of said anilox roll with the surface having irregularities of the paperboard material in rolling engagement with the circumference of said anilox roll and contiguous with said pores whereby the fluid contained within said pores which are engaged by said surface having irregularities is wettingly transferred to the surface having irregularities of said paperboard material.

2. The method of claim 1 including the step of doctoring from the outer circumference surface of said anilox roll substantially all sterilant fluid not disposed within said pores.

3. The method of claim 1 further including the step of providing a pressure roll having its axis parallel to the axis of said anilox roll and in rolling contact with a portion of the outer circumferential surface of said anilox roll containing said pores after said portion of said anilox roll containing said pores has moved through and out of said reservoir of said sterilant fluid, thereby removing substantially all sterilant fluid from the outer circumference of said anilox roll containig said pores that is not disposed within said pores.

4. Apparatus for applying a sterilant fluid to at least one surface of paperboard material, the surface being **characterized by** one or more surface irregularities in the smoothness and/or continuity of the surface comprising:
a) a reservoir for containing a supply of sterilant fluid therein,
b) an anilox roll having at least a portion of its outer circumference partially submerged in said sterilant fluid said anilox roll having defined in the outer circumference thereof a plurality of minute discrete pores opening outward of the the outer circumference of said anilox roll and being spaced apart by respective minimal distances,
c) a doctor blade for removing from the cylindrical surface of said anilox roll excess sterilant over that sterilant contained within said pores,
d) means for rotating said anilox roll while said portion of its outer circumference is partially submerged in said fluid whereby sterilant fluid becomes disposed within said pores,
e) means directing the at least one surface having irregularities of the paperboard material into rolling engagement with said fluid-containing pores of said anilox roll to effect wettingly transfer of fluid from said pores to the surface having irregularities of the paperboard.

5. The apparatus of claim 4 including said doctor blade in sliding contact with the free surface of said anilox roll surface and positioned immediately downstream of the reservoir fluid surface.

6. A paperboard aseptic packaging machine, wherein said paperboard including a surface having one or more surface irregularities in the smoothness and/or continuity of the surface is sterilized using a fluid sterilant prior to forming a package, the machine comprising:
a) a reservoir for containing a supply of sterilant fluid therein,
b) an anilox roll having a multiplicity of porous cavities about the cylindrical surface thereof,
c) means for applying a liquid sterilant upon the cylindrical surface of said anilox roll whereby said pores of said anilox roll are caused to fill with said sterilant,
d) means for removing from the cylindrical surface of said anilox roll excess sterilant over that sterilant contained within said pores,
e) a pressure roll having its axis of rotation parallel to the axis of rotation of said anilox roll and having its cylindrical surface in rolling contact with the cylindrical surface of said anilox roll forming a nip therebetween for receipt therein of a paperboard web whereby the surface having irregularities of said paperboard web passing through said nip and between said pressure roll and said anilox roll is wetted by said fluid sterilant.

7. The apparatus of claim 6, wherein said means for applying said sterilant to said anilox roll comprises spray means whereby said liquid sterilant is sprayed upon the cylindrical surface of said anilox roll.

## Patentansprüche

1. Verfahren zum Sterilisieren eines Kartonmaterials, das eine Oberfläche hat, die durch eine oder mehrere Oberflächenunregelmäßigkeiten hinsichtlich der Glätte und/oder der Durchgängigkeit derselben **gekennzeichnet** ist, wobei es die folgenden Schritte umfasst:
a) Bereitstellen eines Vorratsbehälters mit Desinfektionsmittelfluid,
b) Bereitstellen einer Anilox-Walze, wobei wenigstens ein Teil ihres Außenumfangs wenigstens teilweise in das Fluid eingetaucht ist und im Außenumfang der Anilox-Walze eine Vielzahl kleiner einzelner Poren ausgebildet ist, die durch jeweilige minimale Abstände voneinander entfernt sind,
c) Drehen der Anilox-Walze in dem Fluid, so dass die Poren der Anilox-Walze eine Menge des Fluids darin aufnehmen,
d) Entfernen im Wesentlichen des gesamten Desinfektionsmittelfluids, das sich nicht in den Poren befindet, vom Außenumfang der Anilox-Walze mit einer Rakel,
e) anschließend Leiten des Kartonmaterials über den freien Abschnitt der Anilox-Walze, wobei die Oberfläche, die Unregelmäßigkeiten des Kartonmaterials aufweist, in Rolleingriff mit dem Umfang der Anilox-Walze ist und an den Poren anliegt, so dass das Fluid, das in den Poren enthalten ist, die mit der Oberfläche in Eingriff kommen, die Unregelmäßigkeiten aufweist, benetzend auf die Oberfläche des Kartonmaterials übertragen wird, die Unregelmäßigkeiten aufweist.

2. Verfahren nach Anspruch 1, das den Schritt des Abschabens im Wesentlichen des gesamten Desinfektionsmittelfluids, das sich nicht in den Poren befindet, von der Außenumfangsfläche der Anilox-Walze einschließt.

3. Verfahren nach Anspruch 1, das des Weiteren den Schritt des Bereitstellens einer Druckwalze einschließt, deren Achse parallel zur Achse der Anilox-Walze und in Rollkontakt mit einem Abschnitt der Außenumfangsfläche der Anilox-Walze ist, die die Poren enthält, nachdem sich der Abschnitt der Anilox-Walze, der die Poren enthält, durch den Vorratsbehälter mit dem Desinfektionsmittelfluid hindurch und aus ihm heraus bewegt hat, um so im Wesentlichen das gesamte Desinfektionsmittelfluid, das sich nicht in den Poren befindet, von dem Außenumfang der Anilox-Walze zu entfernen, der die Poren enthält.

4. Vorrichtung zum Auftragen eines Desinfektionsmittelfluids auf wenigstens eine Oberfläche von Kartonmaterial, wobei die Oberfläche durch eine oder mehrere Oberflächenunregelmäßigkeiten bezüglich der Glätte und/oder Durchgängigkeit der Oberfläche **gekennzeichnet** ist, wobei sie umfasst:
a) einen Vorratsbehälter, der einen Vorrat an Desinfektionsmittelfluid enthält,
b) eine Anilox-Walze, wobei wenigstens ein Teil ihres Außenumfangs teilweise in das Desinfektionsmittelfluid eingetaucht ist und im Außenumfang der Anilox-Walze eine Vielzahl kleiner einzelner Poren ausgebildet ist, die sich von dem Außenumfang der Anilox-Walze nach außen öffnen und um jeweilige minimale Abstände voneinander entfernt sind,
c) eine Rakel zum Entfernen von überschüssigem Desinfektionsmittel über das in den Poren enthaltende Desinfektionsmittel hinaus von der zylindrischen Oberfläche der Anilox-Walze,
d) eine Einrichtung, mit der die Anilox-Walze gedreht wird, während der Abschnitt ihres Außenumfangs teilweise in das Fluid eingetaucht ist, so dass das Desinfektionsmittelfluid in den Poren angeordnet wird,
e) eine Einrichtung, mit der wenigstens eine Oberfläche des Kartonmaterials, die Unregelmäßigkeiten aufweist, in Rolleingriff mit den Fluid enthaltenden Poren Anilox-Walze geleitet wird, um benetzende Übertragung von Fluid aus den Poren auf die Oberfläche des Kartons zu bewirken, die Unregelmäßigkeiten aufweist.

5. Vorrichtung nach Anspruch 4, die die Rakel enthält, die in Gleitkontakt mit der freien Fläche der Oberfläche der Anilox-Walze ist und unmittelbar nach der Fluidoberfläche des Vorratsbehälters angeordnet ist.

6. Maschine zum aseptischen Verpacken in Karton, wobei der Karton, der eine Oberfläche enthält, die eine oder mehrere Oberflächenunregelmäßigkeiten bezüglich der Glätte und/oder der Durchgängigkeit der Oberfläche aufweist, unter Verwendung eines Desinfektionsmittelfluids vor Ausbilden einer Verpackung desinfiziert wird und die Maschine umfasst:
a) einen Vorratsbehälter, der einen Vorrat an Desinfektionsmittelfluid enthält,
b) eine Anilox-Walze, die eine Vielzahl poröser Hohlräume um die zylindrische Oberfläche derselben herum aufweist,
c) eine Einrichtung zum Auftragen eines flüssigen Desinfektionsmittels auf die zylindrische Oberfläche der Anilox-Walze, so dass die Poren der Anilox-Walze mit dem Desinfektionsmittel gefüllt werden,
d) eine Einrichtung zum Entfernen von überschüssigem Desinfektionsmittel über das in den Poren enthaltene Desinfektionsmittel hinaus von der zylindrischen Oberfläche der Anilox-Walze,
e) eine Druckwalze, deren Drehachse parallel zu der Drehachse der Anilox-Walze ist und deren zylindrische Oberfläche so in Rolleingriff mit der zylindrischen Oberfläche der Anilox-Walze ist, dass ein Spalt dazwischen ausgebildet wird, um darin eine Kartonbahn aufzunehmen, wobei die Oberfläche der Kartonbahn, die Unregelmäßigkeiten aufweist und durch den Spalt sowie zwischen der Druckwalze und der Anilox-Walze hindurch läuft, mit dem Desinfektionsmittelfluid benetzt wird.

7. Vorrichtung nach Anspruch 6, wobei die Einrichtung zum Auftragen des Desinfektionsmittel auf die Anilox-Walze eine Sprüheinrichtung umfasst, so dass das flüssige Desinfektionsmittel auf die zylindrische Oberfläche der Anilox-Walze gesprüht wird.

## Revendications

1. Procédé pour stériliser un matériau en carton présentant une surface **caractérisée par** une ou plusieurs aspérités et/ou discontinuités, comprenant les étapes qui consistent :
a) à prévoir un réservoir de fluide stérilisateur,
b) à prévoir un rouleau anilox dont une partie au moins de la circonférence extérieure est au moins en partie plongée dans le fluide, une multiplicité de minuscules pores discrets étant définis dans la circonférence extérieure du rouleau anilox et étant espacés les uns des autres par des distances minimales,
c) à faire tourner le rouleau anilox dans le fluide pour que ses pores reçoivent une quantité du fluide,
d) à enlever de la circonférence extérieure du rouleau anilox, à l'aide d'une racle, quasiment tout le fluide stérilisateur qui ne se trouve pas dans les pores,
e) puis à faire passer le matériau en carton sur la partie exposée du rouleau anilox, la surface irrégulière dudit matériau étant en contact par rotation avec la circonférence du rouleau anilox et étant voisine des pores, moyennant quoi le fluide contenu dans les pores qui sont en contact avec la surface irrégulière du matériau en carton est transféré par mouillage sur celle-ci.

2. Procédé selon la revendication 1, comprenant l'étape qui consiste à racler sur la surface de la circonférence extérieure du rouleau anilox quasiment tout le fluide stérilisateur qui ne se trouve pas dans les pores.

3. Procédé selon la revendication 1, comprenant également l'étape qui consiste à prévoir un rouleau presseur dont l'axe est parallèle à l'axe du rouleau anilox et qui est en contact par rotation avec une partie de la surface de la circonférence extérieure dudit rouleau anilox présentant les pores, après que ladite partie du rouleau anilox présentant les pores a traversé le réservoir de fluide stérilisateur et est sortie de celui-ci, ce qui enlève de la circonférence extérieure du rouleau anilox présentant les pores quasiment tout le fluide stérilisateur qui ne se trouve pas dans les pores.

4. Appareil pour appliquer un fluide stérilisateur sur au moins une surface d'un matériau en carton, cette surface étant **caractérisée par** une ou plusieurs aspérités et/ou discontinuités, comprenant :
a) un réservoir qui est destiné à contenir une réserve de fluide stérilisateur,
b) un rouleau anilox dont une partie au moins de la circonférence extérieure est en partie immergée dans le fluide stérilisateur, une multiplicité de minuscules pores discrets étant définis dans la circonférence extérieure du rouleau anilox, s'ouvrant vers l'extérieur de ladite circonférence et étant espacés les uns des autres par des distances minimales,
c) une racle qui est destinée à enlever de la surface cylindrique du rouleau anilox l'excédent d'agent stérilisateur par rapport à l'agent stérilisateur contenu dans les pores,
d) des moyens pour faire tourner le rouleau anilox alors que ladite partie de sa circonférence extérieure est en partie immergée dans le fluide stérilisateur, lequel fluide entre alors dans les pores,
e) des moyens qui mettent ladite surface du matériau en carton présentant des irrégularités en contact par rotation avec les pores du rouleau anilox qui contiennent le fluide, afin d'effectuer un transfert par mouillage du fluide des pores vers ladite surface présentant des irrégularités.

5. Appareil selon la revendication 4, dans lequel la racle est en contact de glissement avec la surface libre du rouleau anilox et est placée directement en aval de la surface du fluide du réservoir.

6. Machine d'emballage aseptique pour carton dans laquelle le carton, qui comprend une surface avec plusieurs aspérités et/ou discontinuités, est stérilisé à l'aide d'un agent stérilisateur avant la mise en forme d'un emballage, cette machine comprenant :
a) un réservoir qui est destiné à contenir une réserve de fluide stérilisateur,
b) un rouleau anilox qui présente sur sa surface cylindrique une multiplicité de cavités en forme de pores,
c) des moyens pour appliquer un agent stérilisateur liquide sur la surface cylindrique du rouleau anilox, moyennant quoi les pores dudit rouleau anilox se remplissent d'agent stérilisateur,
d) des moyens pour enlever de la surface cylindrique du rouleau anilox l'excédent d'agent stérilisateur par rapport à l'agent stérilisateur contenu dans les pores,
e) un rouleau presseur dont l'axe de rotation est parallèle à l'axe de rotation du rouleau anilox et dont la surface cylindrique est en contact par rotation avec la surface cylindrique du rouleau anilox, en formant entre les deux rouleaux un interstice destiné à recevoir une bande de carton, moyennant quoi la surface irrégulière de ladite bande de carton qui passe dans l'interstice et entre le rouleau presseur et le rouleau anilox est mouillée avec l'agent stérilisateur fluide.

7. Appareil selon la revendication 6, dans lequel les moyens pour appliquer l'agent stérilisateur sur le rouleau anilox comprennent des moyens de pulvérisation, le liquide stérilisateur étant pulvérisé sur la surface cylindrique du rouleau anilox.
